(19) Europäisches Patentamt
European Patent Office
Office européen des brevets

(11) Publication number: **0 051 415**
**B1**

(12) # EUROPEAN PATENT SPECIFICATION

(45) Date of publication of patent specification: **14.11.84**

(21) Application number: **81305027.5**

(22) Date of filing: **26.10.81**

(51) Int. Cl.³: **C 07 D 239/95,**
C 07 C 143/10, C 07 C 59/147,
A 61 K 31/505

(54) **Water-soluble salts of 2,4-diamino-5-methyl-6-((3,4,5-trimethoxyanilino)methyl)quinazoline, compositions containing such salts and the production of such salts.**

(30) Priority: **31.10.80 US 202512**

(43) Date of publication of application:
**12.05.82 Bulletin 82/19**

(45) Publication of the grant of the patent:
**14.11.84 Bulletin 84/46**

(84) Designated Contracting States:
**AT BE CH DE FR GB IT LI LU NL SE**

(56) References cited:
**GB-A-1 345 502**

**Chemical Abstracts, Ninth Collective Index, Chemical Substance, vol. 76 to 85, 1972 to 1976 Columbus, Ohio, USA page 15248CS**

(73) Proprietor: **WARNER-LAMBERT COMPANY**
**201 Tabor Road**
**Morris Plains New Jersey 07950 (US)**

(72) Inventor: **Colbry, Norman Lloyd**
**13926 Bramble Brae**
**Gregory Michigan 48137 (US)**

(74) Representative: **Jones, Michael Raymond et al**
**HASELTINE LAKE & CO. 28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to water-soluble salts of 2,4-diamino-5-methyl-6-[(3,4,5-trimethoxy-anilino)methyl]quinazoline, compositions containing such salts and the production of such salts.

The compound 2,4-diamino-5-methyl-6-[(3,4,5-trimethoxyanilino)methyl]quinazoline and its production are reported in British Patent Specification No. 1,345,502, which is incorporated by reference; this compound is reported as exhibiting anti-malarial and anti-bacterial activity. This compound, which has the formula:—

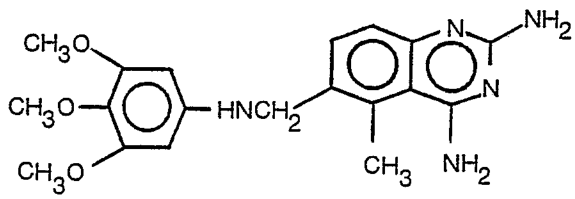

has also been reported to exhibit anti-neoplastic activity in Biochemical Pharmacology *28*, 1983—1987(1978) and Cancer Research *39*, 293—304(1979). Unfortunately, the salts that have previously been studied do not have a high degree of water-solubility. This is especially disadvantageous when treating neoplasms since injectable routes are preferred.

Known salts have been found to be unacceptable for a host of reasons: e.g. lack of solubility, high toxicity, instability, non-crystalline structure, and failure to be recognized as safe by the United States Food and Drug Administration.

One aspect of the present invention provides soluble, non-toxic, stable, crystalline salts having the names 2,4-diamino-5-methyl-6-[(3,4,5-trimethoxyanilino)methyl]-quinazoline monoisethionate and 2,4-diamino-5-methyl-6-[(3,4,5-trimethoxyanilino)methyl]quinazoline glucuronate. The "monoisethionate" salt is the salt of the quinazoline base with the acid, 2-hydroxyethanesulphonic acid, of formula $HO—CH_2—CH_2—SO_3H$.

The salts of the aforementioned aspect of the present invention have the following formula I:—

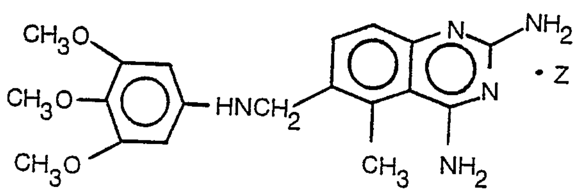

(I)

wherein Z is 2-hydroxyethanesulphonic acid or glucuronic acid. The term "glucuronic acid" as used herein is intended to encompass all the isomeric forms of glucuronic acid with the preferred form being the naturally occurring form of glucuronic acid. Glucuronic acid has the formula $(CHO(CHOH)_4COOH$.

The compounds of formula I wherein Z is 2-hydroxyethanesulphonic acid or glucuronic acid may, in accordance with a further aspect of the present invention, be produced by reacting 2,4-diamino-5-methyl-6-[(3,4,5-trimethoxyanilino)methyl]quinazoline with 2-hydroxyethanesulphonic acid or glucuronic acid, respectively.

The ratio of reactants is not critical and, while equimolar quantitites of reactants may be employed, an excess of acid is generally preferred.

The reaction is preferably carried out in a polar solvent, such as methanol, ethanol, acetone or water, or a mixture thereof. The reaction is preferably carried out at a temperature in the range from 0°C to 100°C (depending on the boiling point of the solvents used) for a period in the range from a few minutes to twenty-four hours. A more preferred range of conditions is from 25°C to 80°C, for about one to eight hours.

It should be noted that the reaction can take place using a suspension of the base in the polar solvent.

The two compounds of the present invention exhibit excellent properties for use as pharmaceutical compounds. The 2,4-diamino-5-methyl-6-[(3,4,5-trimethoxyanilino)-methyl]quinazoline monoisethionate salt is a stable, crystalline, highly water-soluble salt. The 2,4-diamino-5-methyl-6-[(3,4,5-trimethoxyanilino)methyl]quinazoline glucuronate salt appears to be powdery rather than crystalline, but has a higher degree of solubility.

These conclusions are based on the results of experiments reported in the following Tables I and II. Table I reports the results of experiments in which 2,4-diamino-5-methyl-6-[(3,4,5-trimethoxy-

anilino)methyl]quinazoline was added to an aqueous acidic solution, while Table II reports the solubility in water of prepared 2,4-diamino-5-methyl-6-[(3,4,5-trimethoxyanilino)methyl]quinazoline salts. A comparison of the results obtained from the two methods is reported in the following Table III.

Most salts were found to have an unsatisfactory degree of solubility in water, such as the 2-naphthalene sulphonate; or an undesirable amorphous form, such as the gluconate or galacturonate; or were not acceptable because extensive studies to determine safety would have had to be performed, such as ethoxyacetic acid.

The compounds of the present invention are useful in treating malaria or as an anti-bacterial in mammals, such as dogs, cats or cattle, conveniently in dosage unit form with the dose adjusted to the needs and tolerances of the subject being treated. The usual mammalian dosage range for a 70 kg subject is from 3.5 to 350 mg per day (0.05 mg to 5.0 per kg of body weight per day), preferably 7.0 mg to 140 mg per day (0.1 mg to 2.0 mg per kg of body weight per day).

TABLE I

The solubility of 2,4-diamino-5-methyl-6-[(3,4,5-trimethoxyanilino)methyl]quinazoline in aqueous acids

| Aqueous Acid | pH. 1*M*. soln. | Solubility of base in acid soln. |
|---|---|---|
| Glucuronic | 2.05 | 34.35 (mg base/ml soln.) |
| Gluconic | 2.27* | 19.8 |
| Ethoxyacetic | 2.20 | 19.5 |
| Galacturonic | 2.28 | 25.1 |
| Etoxyacetic | 2.35* | 19.7 |
| Isethionic | 1.20 | 16.3 |
| Isethionic | 1.46* | 17.3 |
| *N*-(Morpholine)ethane-2-sulphonic | 1.52 | 11.2 |
| Methane sulphonic | 1.00 | 9.83 |
| Methane sulphonic | 1.42* | 6.49 |
| Citric | 1.95 | 8.28 |
| Benzene sulphonic | 0.95 | 7.17 |
| Benzene sulphonic | 1.48* | 1.89 |
| Glycolic | 3.00 | 4.13 |
| Glycolic | 3.28* | 4.73 |
| *p*-Toluene sulphonic | 1.45 | 2.74 |
| d-Tartaric | 1.82 | 1.57 |
| (2-Pyridyl)-2-ethane sulphonic | 3.35 | 1.73 |
| 2-Amino-ethane sulphonic | 4.28 | .51 |
| 2-Naphthalene sulphonic | 1.05 | .15 max. |

*pH of .05 *M* acid-test method using 1/1 acid/base

Method: 10.0 ml aliquots of 0.1 *M* acid solutions were prepared; then 185 $\pm$ 3 mg of 2,4-diamino-5-methyl-6-[(3,4,5-trimethoxyanilino)methyl]quinazoline were added (0.05 mole of base); the

resulting mixture was shaken for from 4 to 12 hours, filtered, and 1.0 ml of filtrate diluted with 0.01 *N* HCl to read UV absorbance at 321 nm.

TABLE II

Solubility of 2,4-diamino-5-methyl-6-[(3,4,5-trimethoxyanilino)-methyl]quinazoline salts in water

| Salt | Method | Solubility mg base/ml |
|---|---|---|
| Glucuronate | B | 109.4 |
| Isethionate | B | 24.78 |
| Isethionate | A | 23.93 |
| Ethoxyacetate | B | 6.3 |
| 3-Hydroxypropyl sulphonate | B | 4.73 |
| Lactate | A | 4.34 |
| Hydrochloride | A | 3.22 |
| Gluconate | B | 2.55 |
| Ethane sulphonate | B | 2.64 |
| Acetate | A | 1.72 |
| Sulphate | A | 1.61 |
| Gentisate | A | 1.4—7.06* |
| Gentisate | B | 0.28 |
| Benzene sulphonate | B | 1.24 |
| *Controls:* | | |
| Free base — 2,4-diamino-5-methyl-6-[(3,4,5-trimethoxyanilino) methyl]quinazoline | A | 0.2 |
| Free base — 2,4-diamino-5-methyl-6-[(3,4,5-trimethoxyanilino) methyl]quinazoline | B | 0.1 |

* Analytical sample preparation questionable.

Method A: 2.0 ml $H_2O$ were saturated with salt overnight; 0.5 ml of filtrate was diluted to 10.0 ml with methanol, and 1 drop of 6*N* KOH added to UV cell.

Method B: 5.0 ml $H_2O$ were saturated with salt overnight; 1.0 ml of filtrate was diluted with .01 *N* HCl (10 to 250 fold dilutions).

TABLE III
Method Comparison — Selected 2,4-diamino-5-methyl-6-[(3,4,5-trimethoxyanilino)-methyl]quinazoline salts

| Acid | Acid Conc. (M) | Acid pH | Acid Base Molar Ratio | Solubility mg base/ml | Filtrate pH |
|---|---|---|---|---|---|
| Isethionic | .1 | 1.20 | 2/1 | 16.33* | 1.83 |
| | .05 | 1.46 | 1/1 | 17.34* | 3.70 |
| | Prepared Salt | | | 24.8 | 5.30 |
| Ethoxyacetic | .1 | 2.20 | 2/1 | 19.5* | |
| | .065 | 2.35 | 1/1 | 19.7* | 4.15 |
| | Prepared Salt | | | 6.3 | 5.32 |
| Methane sulphonic | .1 | 1.00 | 1/.9 | 8.74 | |
| | .06 | 1.42 | 1/1 | 6.5 | 3.00 |
| | Prepared Salt | | | 4.9 | 5.32 |
| Benzene Sulphonic | .1 | 0.95 | 2/1 | 7.17 | 2.95 |
| | .05 | 1.48 | 1/1 | 1.89 | 2.95 |
| | Prepared Salt | | | 1.24 | 4.45 |
| Glycolic | .1 | 2.68 | 2/1 | 4.13 | 3.00 |
| | .1 | 2.69 | 1/1 | 4.73 | 3.28 |
| | Prepared Salt | | | 2.55 | 5.30 |

*Not saturated, base solubility equals amount of base available in shake out preparation.

The novel salts of the present invention may be administered orally, parenterally or rectally.

Another aspect of the present invention provides a pharmaceutical composition comprising a therapeutically acceptable amount of a novel compound according to the present invention, and a pharmaceutically acceptable carrier.

In accordance with the present invention, oral pharmaceutical compositions may be produced by formulating the novel compounds of the present invention, as the active ingredient, in dosage unit forms with a pharmaceutical carrier. Some examples of suitable dosage unit forms are tablets, capsules, lozenges and pills; as well as powders and aqueous and non-aqueous solutions and suspensions packaged in containers containing either one or some larger number of dosage units and capable of being sub-divided into individual doses by such means as measurement into a teaspoon or other standard container. Some examples of suitable pharmaceutical carriers, including pharmaceutical diluents, are gelatin capsules; sugars such as lactose and sucrose; starches such as corn starch and potato starch; cellulose derivatives such as sodium carboxymethyl cellulose, ethyl cellulose, methyl cellulose and cellulose acetate phthalate; gelatin; talc; stearic acid; magnesium stearate; vegetable oils such as peanut oil, cottonseed oil, sesame oil, olive oil, corn oil, and oil of theobroma; propylene glycol; glycerin; sorbitol; polyethylene glycol; water; agar; alginic acid; as well as other compatible substances normally used in pharmaceutical formulations. The pharmaceutical compositions of the present invention can also contain other components such as colouring agents, flavouring agents, and/or preservatives. These materials, if present, are usually used in relatively small amounts. The compositions can, if desired, also contain other therapeutic agents.

The novel compounds of the present invention when intended for parenteral use could be dissolved in an isotonic aqueous solution containing other materials such as buffers or preservatives. The novel compounds may also be placed in the form of an sterile lyophilized material to be taken up in an appropriate vehicle at time of use.

Further, the compounds of the present invention may be administered in the form of a suppository using, for instance, glycerin or cocoa butter as a vehicle. The vehicle may also contain preservatives and colouring agents.

The percentage of the active ingredient in the foregoing compositions can be varied within wide limits but for practical purposes it is preferably present in a concentration of at least 10% in a solid composition and at least 2% in a primarily liquid composition. The most satisfactory compositions are those in which a much higher proportion of the active ingredient is present. The compositions of the invention preferably contain from 1 to 100 mg, preferably from 2 to 50 mg, of the active ingredient per dosage unit so that the entire amount to be administered during a day can be made up from a reasonable number of dosage units.

The compounds of the present invention can be combined with other anti-malarial compounds,

such as quinine or chloroquine, and used in the above-described manner.

The compounds of the present invention are also useful in treating neoplasms and psoriasis in mammals. While the compositions may be formulated in the same manner that anti-malarial composiions are used, the preferred route of administration is the parenteral route, especially intravenously. The usual mammalian dosage range, for treating neoplasms or psoriasis, for a 70 mg human subject is from 5 to 500 mg per day (0.07 mg to 7.1 mg per kg of weight per day), preferably 15 to 120 mg per day (0.2 mg to 1.7 mg per kg of weight per day), optionally in divided portions. Therapeutic agents of this type are generally prescribed for a specific time span, such as for a five day period depending upon the disease state being treated.

Thus choriocarcinoma type states may be treated with 15 mg to 60 mg per day for a five day period and repeated three to five times as required while mycosis fungoides type condition may require lower doses, such as 5 mg to 25 mg daily over greater periods of time which may be measured in weeks or months.

In addition, the compounds of this invention may be employed in combination or conjunction with other agents, such as cyclophosphamide, vincristine, cytarabine, fluorouracil, mercaptopurine, prednisone or lomustine. When used in combination or conjunction with the foregoing agents, the dosage may be lowered.

The compounds of the present invention inhibit the growth of pathogenic bacteria, such as *Streptococcus faecalis* (MGH—s), normal (UC—76) and drug-resistant (S18713); *Staphylococcus aureus*; *Escherichia coli* (Vogel) and and *Shigella sonnei* (C—10). Thus, the compounds of the present invention would be useful in, for example, the sterilization of laboratory glassware.

*The production of 2-hydroxyethanesulphonic acid solution*

A solution of 10 g of 2-hydroxyethanesulphonic acid, sodium salt in 30 ml of $H_2O$ was eluted through 120 g of an acid-form ion exchange column Dowex 50WX8 (DOW Chemical Co., Midland, Michigan, United States of America). The resulting acidic solution (200 ml) was titrated and found to be 0.304 molar in the desired acid.

The following Examples illustrate the preparation of the novel salts of the present invention.

Example 1

*2,4-diamino-5-methyl-6-[(3,4,5-trimethoxyanilino)methyl]quinazoline monoisethionate*

A slurry of 2,4-diamino-5-methyl-6-[(3,4,5-trimethoxyanilino)methyl]quinazoline monoacetate (59.2 g) (as described in British Patent Specification No. 1,345,502) in water (1.5 l) at 80°C was treated with acetic acid (200 ml). The resulting solution was made basic (pH 10) by the addition of 50% NaOH solution. The slurry was then cooled by addition of ice and the solid collected. Drying (100°C, 1 torr, 2 hours) gave the free base as a light green-yellow powder.

A slurry of the free base (22.9 g) in acetone (1 l) was treated with 0.304 *M* aqueous 2-hydroxyethanesulphonic acid (204 ml), then cooled and filtered to remove a small amount of solid. The filtrate was concentrated to a solid which was recrystallized from a mixture of ethanol (400 ml) and acetone (400 ml) and dried (80°C, 1 torr, 4 hours) to give the title compound, mp 189—191°C.

Example 2

*2,4-diamino-5-methyl-6-[(3,4,5-trimethoxyanilino)methyl]quinazoline D-glucuronate*

A mixture of 2,4-diamino-5-methyl-6-[(3,4,5-trimethoxyanilino)methyl]quinazoline (1.0 g) and glucuronic acid (0.7 g) in methanol (65 ml) was heated to dissolve the solid. The resulting solution was cooled to 10°C and filtered to remove a small amount of solid. The filtrate was heated to reflux and ethyl acetate was added to the cloud point. The warm solution was filtered and then slowly cooled. The solid that formed was collected, washed first with ethyl acetate and then with diethyl ether, and then dried *in vacuo* at 60°C to give the salt as a yellow powder with no definite melting point.

**Claims for the Contracting States: BE CH DE FR GB IT LI LU NL SE**

1. A compound having the following formula:

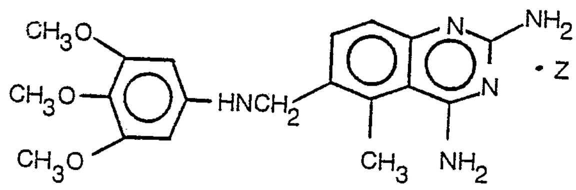

wherein Z is 2-hydroxyethanesulphonic acid or is glucuronic acid.

2. The compound of Claim 1 being 2,4-diamino-5-methyl-6-[(3,4,5-trimethoxyanilino)-methyl]quinazoline monoisoethionate.

3. The compound of Claim 1 being 2,4-diamino-5-methyl-6-[(3,4,5-trimethoxyanilino)-methyl]-quinazoline glucuronate.

4. A pharmaceutical composition comprising a therapeutically acceptable amount of a compound of Claim 1 and a pharmaceutically acceptable carrier.

5. A process for preparing a compound having the following formula:

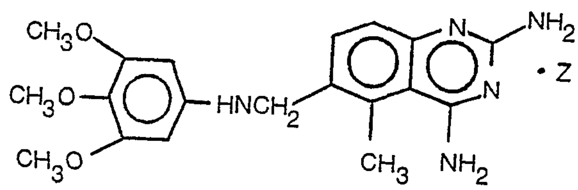

wherein Z is as defined in Claim 1, which process comprises reacting a compound of the following formula:

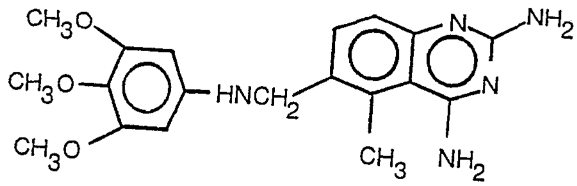

with 2-hydroxethanesulphonic acid or with glucuronic acid.

**Claims for the Contracting State: AT**

1. A process for preparing a compound having the following formula I:

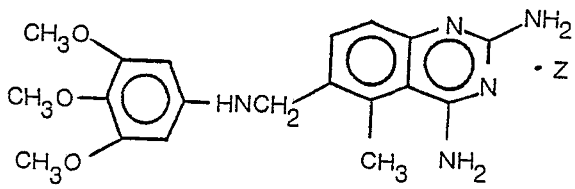

(I)

wherein Z is 2-hydroxyethanesulphonic acid or glucuronic acid, which process comprises reacting a compound of the following formula:

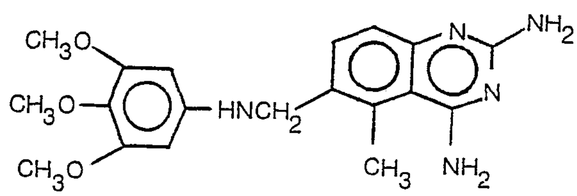

with 2-hydroxyethanesulphonic acid or with glucuronic acid.

2. A process according to Claim 1, for producing the compound being 2,4-diamino-5-methyl-6-[(3,4,5-trimethoxyanilino)methyl]quinazoline monoisethionate.

3. A process according to Claim 1, for producing the compound being 2,4-diamino-5-methyl-6-[(3,4,5-trimethoxyanilino)methyl]quinazoline glucuronate.

4. A process for producing a pharmaceutical composition comprising a therapeutically acceptable amount of a compound of formula I as shown in Claim 1, which comprises incorporating that compound in a pharmaceutically acceptable carrier.

**Patentansprüche für die Vertragsstaaten: BE CH DE FR GB IT LI LU NL SE**

1. Verbindung der folgenden Formel

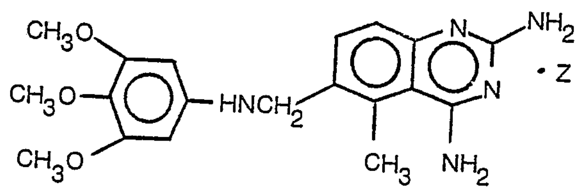

worin Z 2-Hydroxyäthansulfonsäure oder Glucuronsäure bedeutet.

2. Verbindung nach Anspruch 1, welche 2,4-Diamino-5-methyl-6-[3,4,5-trimethoxyanilino)-methyl]-chinazolin-monoisethionat ist.

3. Verbindung nach Anspruch 1, welche 2,4-Diamino-5-methyl-6-[(3,4,5-trimethoxyanilino)-methyl]-chinazolin-glucuronat ist.

4. Pharmazeutische Zusammensetzung umfassend eine therapeutisch akzeptable Menge einer Verbindung des Anspruchs 1 und einen pharmazeutisch akzeptablen Träger.

5. Verfahren zur Herstellung einer Verbindung der folgenden Formel:

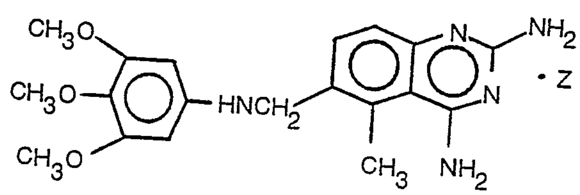

worin Z die in Anspruch 1 angegebene Bedeutung hat, welches Verfahren dadurch gekennzeichnet ist, daß eine Verbindung der folgenden Formel

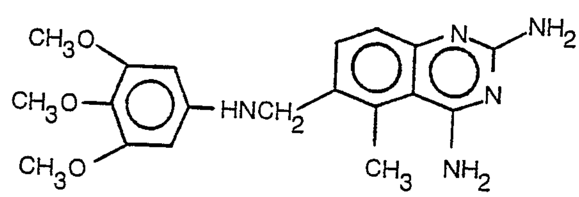

mit 2-Hydroxyäthansulfonsäure oder mit Glucuronsäure umgesezt wird.

**Patentansprüche für den Vertragsstaat: AT**

1. Verfahren zur Herstellung einer Verbindung mit der folgenden Formel I

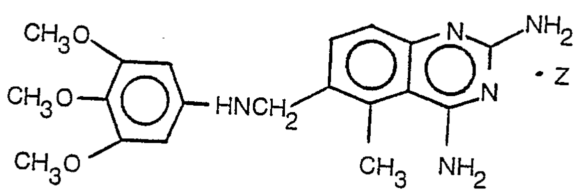

(I)

worin Z 2-Hydroxyäthansulfonsäure oder Glucuronsäure bedeutet, welches Verfahren dadurch gekennzeichnet ist, daß eine Verbindung der folgenden Formel

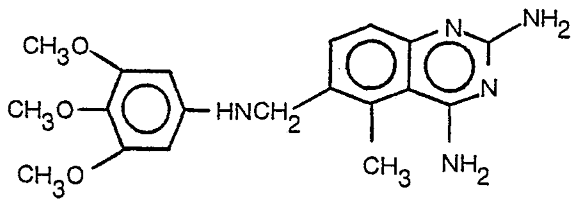

mit 2-Hydroxyäthansulfonsäure oder mit Glucuronsäure umgesetzt wird.

2. Verfahren nach Anspruch 1 zur Herstellung der Verbindung 2,4-Diamino-5-methyl-6-[(3,4,5-Trimethoxyanilino)-methyl]-chinazolin-monoisethionat.

3. Verfahren nach Anspruch 1 zur Herstellung der Verbindung 2,4-Diamino-5-methyl-6-[(3,4,5-trimethoxyanilino)-methyl]-chinazolin-glucuronat.

4. Verfahren zur Herstellung einer pharmazeutischen Zusammensetung umfassend eine therapeutisch akzeptable Menge einer Verbindung der Formel I, wie in Anspruch 1 gezeigt, dadurch gekennzeichnet, daß diese Verbindung in einen pharmazeutisch akzeptablen Träger inkorporiert wird.

**Revendications pour les Etats Contractants: BE CH DE FR GB IT LI LU NL SE**

1. Un composé ayant la formule suivante:

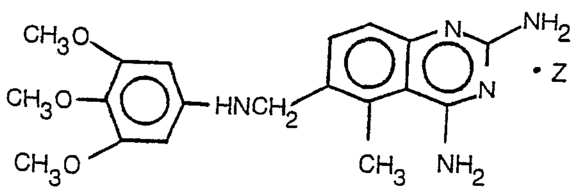

dans laquelle Z est l'acide 2-hydroxyéthanesulfonique ou l'acide glucuronique.

2. Le composé selon la revendication 1, consistant en le monoiséthionate de 2,4-diamino-5-méthyl-6-[(3,4,5-triméthoxyanilino)méthyl]quinazoline.

3. Le composé selon la revendication 1, consistant en le glucuronate de 2,4-diamino-5-méthyl-6-[(3,4,5-triméthoxyanilino)méthyl]quinazoline.

4. Une composition pharmaceutique comprenant une quantité acceptable de point de vue thérapeutique d'un composé selon la revendication 1 et un véhicule pharmaceutiquement acceptable.

5. Un procédé de préparation d'un composé ayant la formule suivante:

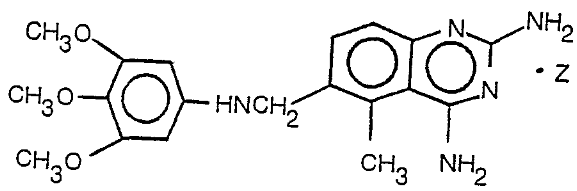

dans laquelle Z est tel que défini dans la revendication 1, lequel procédé comprend la réaction d'un composé ayant la formule suivante avec l'acide 2-hydroxyéthanesulfonique ou l'acide glucuronique.

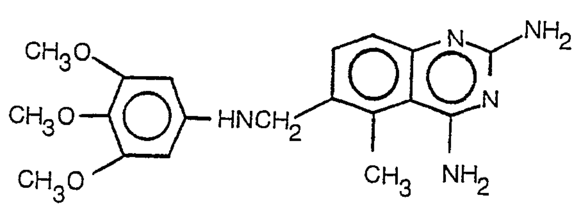

**Revendications pour l'Etat Contractant: AT**

1. Un procédé de préparation d'un composé présentant la formule suivante:

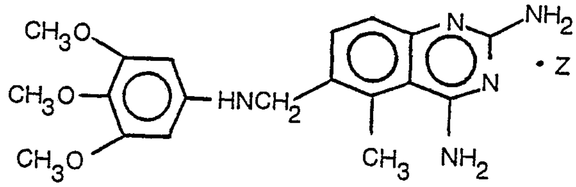

(I)

dans laquelle Z est l'acide 2-hydroxyéthanesulfonique ou l'acide glucuronique, lequel procédé consiste à faire réagir un composé ayant la formule suivante:

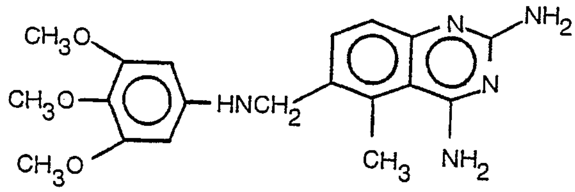

avec l'acide 2-hydroxyéthanesulfonique ou l'acide glucuronique.

2. Un procédé selon la revendication 1, de production de composé consistant en le monoiséthionate de 2,4-diamino-5-méthyl-6-[(3,4,5-triméthoxyanilino)méthyl]quinazoline.

3. Un procédé selon la revendication 1, de production du composé consistant en le glucuronate de 2,4-diamino-5-méthyl-6-[(3,4,5-triméthoxyanilino)méthyl]quinazoline.

4. Un procédé de préparation d'une composition pharmaceutique comprenant une quantité acceptable du point de vue thérapeutique d'un composé de formule (I) tel que montré dans la revendication, qui consiste à incorporer ce composé dans un véhicule pharmaceutiquement acceptable.